(19) Europäisches Patentamt
European Patent Office
Office européen des brevets

(11) EP 1 023 072 B1

(12) **EUROPEAN PATENT SPECIFICATION**

(45) Date of publication and mention of the grant of the patent:
**11.12.2002 Bulletin 2002/50**

(21) Application number: **98949092.5**

(22) Date of filing: **12.10.1998**

(51) Int Cl.$^7$: **A61K 31/55**, A61K 9/107, A61K 9/20 // A61K31:55,(A61K31/485, A61P25:04)

(86) International application number:
**PCT/GB98/03076**

(87) International publication number:
**WO 99/018967 (22.04.1999 Gazette 1999/16)**

(54) **ANALGESIC COMPOSITIONS COMPRISING A CHOLECYSTOKININ AND AN OPIOID**

ANALGETIKA, EINEN CHOLEZYSTOKININ ANTAGONISTEN UND EIN OPIOID ENTHALTEND

COMPOSITIONS ANALGESIQUES COMPRENANT UN ANTAGONISTE DE CHOLECYSTOKININE UN OPIOIDE

(84) Designated Contracting States:
**AT BE CH CY DE DK ES FI FR GB GR IE IT LI LU MC NL PT SE**

(30) Priority: **15.10.1997 GB 9721746**

(43) Date of publication of application:
**02.08.2000 Bulletin 2000/31**

(73) Proprietor: **Panos Therapeutics Limited**
**Little Milton, Oxford OX44 7PU (GB)**

(72) Inventor: **IVERSEN, Leslie, Lars**
**Little Milton Oxford OX44 7PU (GB)**

(74) Representative:
**Sanderson, Laurence Andrew et al**
**SANDERSON & CO.**
**34, East Stockwell Street**
**Colchester Essex CO1 1ST (GB)**

(56) References cited:
**EP-A- 0 222 614**
**EP-A- 0 391 369**
**GB-A- 1 564 039**
**US-A- 4 791 215**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

The present invention relates to pharmaceutical formulations suitable for treating pain, in particular, neuropathic pain and/or dysesthesia, and their preparation. In particular, the present invention relates to formulations comprising a cholecystokinin antagonist and an opioid.

Cholecystokinin (hereinafter 'CCK') has been implicated in a variety of physiological functions, one of which is the control of pain. CCK has been shown to have a heterogeneous distribution within the brain, with the greatest levels being found in the hippocampus, cerebral cortex, amygdala and olfactory lobes. The physiological role of central CCK receptors is still under investigation, but it has many of the features of a neurotransmitter. CCK has been found in regions of the brain known to be associated with pain modulation. Furthermore, mole-per-mole, CCK has been found to be much more potent than morphine in tests for analgesia.

However, at variance with these findings, are results of tests which imply that CCK may antagonise endogenous opiate action. (Faris et al. in Science 219 310-2 (1983)). There is evidence that exogenous CCK attenuates analgesia induced by morphine or release of endogenous opioids. These disparate findings and others imply that large doses of CCK induce a 'pharmacological' analgesia whereas small doses of the peptide produce physiological antagonism of opioid analagesics.

CCK also appears to play a role in the development of tolerance to opioid analgesia as blockade of CCK receptors has been shown to prevent tolerance to morphine. Hence, blockade of CCK receptors by CCK antagonists may reverse or prevent the development of opiate tolerance in patients, and also potentiates the analgesic effects of opioids. The present invention is therefore based on the thesis that blockage of CCK action may be an effective supplement to morphine (or other opioid) administration in the treatment of chronic pain. However, it is believed that this opioid facilitation is preferentially mediated by the central CCK type B receptors since CCK-Bantagonists seem to potentiate the analgesic-effects of both-opioids and non-opioids at the spinal level. Furthermore, facilitation of opiate-induced analgesia by CCK-B receptor antagonists seem to be restricted to μ-, rather than d-, opioid receptor-mediated antinociception. Such μ-opioid againsts include morphine and hydroxymethyl fentanyl. However, potentiation of the analgesic effects produced by these opioids has also been observed with (relatively higher doses of) a CCK-A antagonist.

The present invention therefore generally relates to pharmaceutical formulations comprising an opioid-potentiating amount of a CCK antagonist together with an analgesic amount of an opioid. However, although the most popularly-used opioids such as morphine are not difficult to formulate, particularly for administration by injection, as they are water-soluble drugs, many CCK antagonists, particularly the preferred CCK antagonists to which this invention relates, are relatively insoluble compounds which are therefore pharmaceutically incompatible with hitherto-known formulations of opiate drugs. Having therefore taken the step of appreciating the advantages to be gained by co-administration of an opioid with a CCK antagonist in a single formulation, it was then realised that such a formulation, or a satisfactory carrier for the combination of active ingredients, was not available.

An example of a dual formulation of an opiate analgesic (propoxyphene) with benzodiazepines, is disclosed in British Publication No. 1 564 039. However, the benzodiazepines in question are tranquillisers rather then CCK antagonists, and the pharmacological mechanisms involved are therefore quite different.

European Publication No. 0 222 614 discloses a hydrophobic carrier having a hydrophilic phase to promote the release of a single water-soluble drug, whilst European Publication No. 0 391 369 discloses the use of an oil-in-water emulsion for the delivery of a water-insoluble drug.

However, none of these prior art systems teach a two-phase carrier capable of delivering both a hydrophilic drug and a hydrophobic drug simultaneously.

The present invention is therefore directed at solving this problem and provides a pharmaceutical formulation comprising

(a) an opioid-potentiating amount of a CCK antagonist;
(b) an analgesic amount of an opioid; and
(c) a pharmaceutically acceptable biphasic carrier comprising

    (i) an organic phase comprising a glyceride derivative; and
    (ii) a hydrophilic phase.

The organic phase may be either solid or liquid at room temperature but preferably has a solubilising capacity for the CCK antagonist in excess of 5mg per gram of organic phase. Examples are oils comprising a glyceride which is liquid at room temperature and glyceride waxes having melting points in the range 35-80°C.

The organic phase may therefore comprise, for example, soya bean, safflower, sesame, rapeseed, peanut, olive, cotton seed or fish oils. Preferably, soya bean and/or safflower oils are chosen, alone or in combination with

glycerine. Alternatively, the organic phase may comprise waxes such as full and/or partial glycerides of fatty acids. Preferably, such waxes are triglycerides and partial glycerides of unsaturated $C_{12-18}$ fatty acids such as, for example, Witepsol H15 or W25.

The hydrophilic phase may itself be aqueous, or may be anhydrous but take in and/or dissolve in water in vivo. In the case of formulations for intravenous use, the hydrophilic phase preferably has a viscosity of from 2500-7500cp (2% aqueous at 20°C), more preferably around 4000cp. Such ingredients may also be added to prevent or reduce coalescence of oily droplets of the organic phase. In the case of solid formulations such as tablets and suppositories, the hydrophilic phase is gel-forming and incorporates the opioid in the gel, and also forms a matrix for incorporating the CCK antagonist plus glyceride. The hydrophilic or aqueous phase may therefore comprise a pharmacologically and pharmaceutically acceptable polymer or salts thereof which may be selected from proteins such as gelatine, hyaluronic acid, alginic acids or salts thereof such as sodium alginate, carboxymethylcellulose (optionally cross-linked), methyl cellulose, other cellulose derivatives which are water-swellable such as hydroxypropylmethylcellulose and hydroxyethyl cellulose or other water-swellable polymers such as polyvinyl pyrrolidone (PVP) or water-soluble polymers such as lactose.

In the formulation, the organic and hydrophilic phases may be separated or may be combined, for example, to form an oil-in-water emulsion. Preferred such emulsions therefore comprise:

(i) an oil phase comprising a glyceride derivative; and
(ii) an aqueous phase optionally comprising a buffer whereby the emulsion has a pH of from 6.5 to 7.5 and optionally comprising an isotonicity regulator whereby the aqueous phase is made isotonic to blood plasma.

When the carrier is in the form of an emulsion, the average particle size of the resultant emulsion is preferably in the range 0.2 to 3.0μm, more preferably around 1μm.

Optionally, an emulsifying agent and/or surfactant may be incorporated into the carrier. A suitable surfactant is a sorbitan derivative such as the polysorbates, for example polysorbate 80, or sorbitan monooleate, and the poloxamers such as Pluronic F38. Suitable emulsifying agents include egg yolk lecithin, egg yolk phospholipids such as phosphatidyl choline and the like.

To adjust the pH, a suitable pH adjuster (i.e. an acidifying or alkalising agent) is used such as hydrochloric acid or sodium hydroxide, or a buffer such as a phosphate buffer system. Preferably, the pH is adjusted to 7-7.5, more preferably, it is close to neutral (pH = 7).

Liquid formulations may also comprise an isotonicity regulator to ensure that the aqueous phase thereof is or remains isotonic to blood plasma. Examples of such isotonicity regulators include dextrose, glucose, mannitol, sorbitol, glycerol and sodium chloride.

Other hydrophobic or hydrophilic components may be included in the formulation such as, particularly in the case of suppositories, a thickener or gelling agent for the organic phase such as hydrophobic silicon dioxide or silica; lubricants, particularly in the case of tablet formulations, such as magnesium stearate, stearic acid, talc and LUBITROL, preferably magnesium stearate. Optional other ingredients include colouring or flavouring agents, release agents, pore-forming agents, stabilisers, and fillers or diluents such as lactose, calcium phosphate or carbonate, microcrystalline cellulose and the like, and antioxidants.

Also, in the case of tablets, a coating may be applied such as waxes, fatty alcohols, water-insoluble cellulose derivatives, other water-insoluble polymers such as polymers or copolymers of acrylates and/or methacrylates (eg. EUDRAGIT), ethylcellulose, cellulose acetate, shellac, hydrogenated vegetable oils and the like. Such a coating may provide the mechanism to enable controlled release of the opioid. Such a coating may optionally include a plasticizer or film enhancer such as monoglycerides, phthalates, sebacates, citrates, castor oil and the like.

Preferably, the CCK antagonist is incorporated into the organic phase, and more preferably into the glyceride derivative, and the opioid analgesic is incorporated into the hydrophilic phase. However, the present invention does not preclude having components (a) and (b) present in any combination in any phase of the carrier.

The components are preferably present within the following ranges of ratios: 10:1 to 1:5, respectively (i):(ii); and 1:2 to 1:40, respectively (a):(b).

The opiate drug may be selected from those which are effective analgesics and particularly those which need to be administered at relatively high or increasing doses. Examples include morphine, or a salt thereof such as the sulphate, chloride or hydrochloride, or other 14-hydroxymorphinan opioid analgesics such as naloxone, meperidine, butorphanol or pentazocine, or morphine-6-glucuronide, codeine, dihydrocodeine, diamorphine, dextropropoxyphene, pethidine or fentanyl, or a salt of any of these.

The CCK antagonist may be selected from any of those which potentiate the analgesic effects of the opioid chosen and/or which reverse or prevent patient tolerance thereto. For example, CCK antagonists include those of formulae (I)-(IV) which are defined, respectively, in (I) US 4791 215; (II) EPS 167 919 and 284 256; (III) EP 405 537; and (IV) J. Med. Chem. 34 1508 (1991), which are herein incorporated by reference in their entirety.

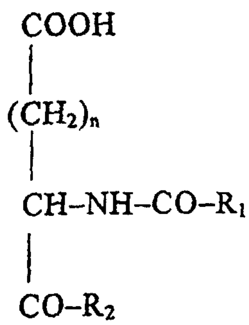

(Ia)

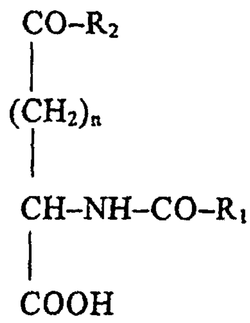

(Ib)

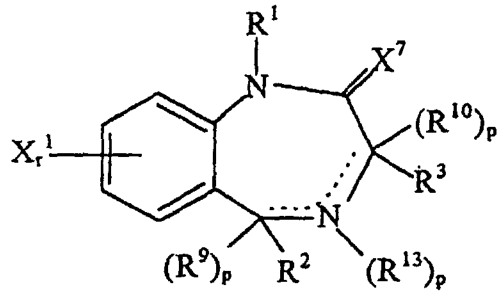

(II)

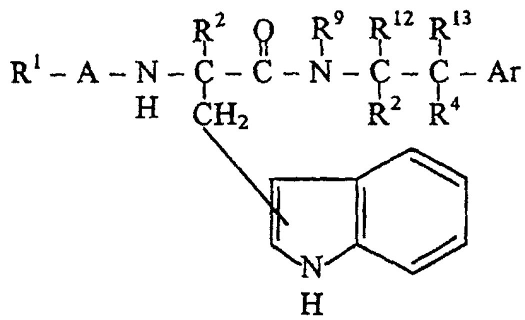

(III)

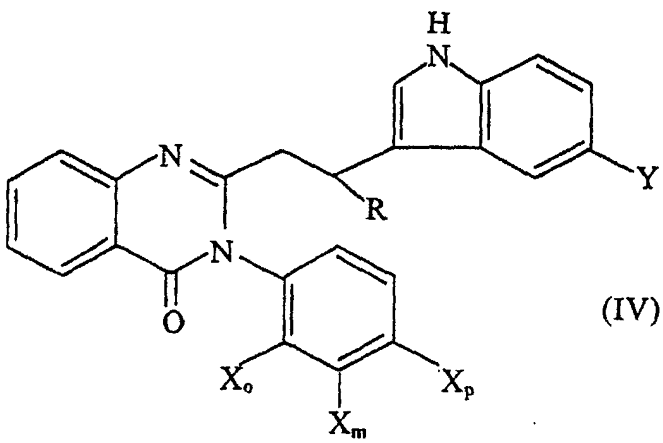

(IV)

Preferred CCK antagonists are selected from those described in European patents specifications nos. 167 919; 284 256; 508 796; 652 871; 411 668; 421 802; and 617 621, which are herein incorporated by reference in their entirety. Particularly preferred CCK antagonists include devazepide (also known as MK-329), namely, 3S-(-)-(1,3-dihydro-3-(2-indolecarbonylamino)-1-methyl-5-phenyl-2H-1,4-benzodiazepin-2-one (a CCK-A antagonist); L-365,260, namely 3R-3-(N-(3-methylphenyl)ureido)-1,3-dihydro-1-methyl-5-phenyl-2H-1,4-benzodiazepin-2-one (a CCK-B antagonist); and so-called second generation compounds such as L-369,466, namely N-[1,3-dihydro-1-methyl-2-oxo-5-phenyl-1H-1,4-benzodiazepin-3-yl]-N'-[3-(1,2,4-oxodiazol-5-one)phenyl]urea, L-741,528, namely (-)-N-[2,3-dihydro-5-(4,4-dimethylpiperidin-1-yl)-1-methyl-2-oxo-1H-1,4-benzodiazepin-3-yl]-N'-[indan-5-yl]urea and [N-[(3R)-5-(3-azabicyclo [3,2,2]nonan-3-yl)-2,3-dihydro-1-methyl-2-oxo-1H-1,4-benzodiazepin-3-yl]-N'-(3-methylphenyl) urea] (L-740,093, a CCK-B antagonist described in Molecular Pharmacol. 46 943-8 (1994)). Especially preferred are the CCK-B antagonists, in particular L-365,260 and the previously-mentioned second generation compounds.

The formulations of the present invention are preferably sustained-, slow- or continuous-release (S.R.) solid formulations, or emulsions for injection. S.R. formulations may be in the form of a suppository or tablet, for example.

The formulations of the present invention are particularly suitable for treating chronic and neuropathic pain. Nerve damage arising from either trauma or disease affecting peripheral nerves leads to abnormal pain states referred to as neuropathic pain. Such pain may be long-lasting and continue for extended periods after the initial injury has healed. Individuals afflicted with neuropathic pain show a marked sensitivity to nociceptive stimuli, indicative of a lowered nociceptive threshold (hyperalgesia). Moreover, there is also a perception of normally innocuous stimuli being nociceptive, a state referred to as allodynia.

In particular, these formulations are suitable for treating patients with spinal cord injury. They prevent tolerance to the opioid analgesic and eliminate the need to increase doses of opioid to clinically unacceptable levels. However, they are also useful in enhancing opioid analgesia in non-pathological pain states, and the anxiolytic (anti-anxiety or anti-panic) effects of some CCK antagonists is a particularly beneficial additional effect.

A suitable daily dose or CCK antagonist in these formulations would preferably be in the range 0.5 to 300mg per day, such as 1 to 100mg/day (oral or via suppository) or 1 to 300mg/day (i.v.). Preferably, for MK-329, the dose would be 1-10mg/day (5-10 mg/day orally or 1-3mg/day i.v.); for L-365,260, the dose would be 10-100mg/day orally (5-10mg/day orally or 10-300 mg/day i.v.); and for 'second generation' CCK antagonists, 1-2mg/day (oral) or 0.5-1.5 mg/day (i.v.).

The present invention will now be illustrated by the following nonlimiting examples:

Example 1 : Intravenous Emulsions

| | | |
|---|---|---|
| (a) | L-740,093 (active (a))<br>Morphine sulphate (active (b))<br>Soya bean oil (i)<br>Phosphatidyl choline (emulsifier)<br>Pluronic F 68 (surfactant)<br>Water (ii)(adjusted to pH 7 to 7.5 and made isotonic with sorbitol q.s.) | 0.00025g<br>0.010g<br>0.4000ml<br>0.0240g<br>0.0040g<br>2ml |

The injection is prepared using aseptic techniques and sterile materials. The MK-329 is dispersed in the soya bean oil and the morphine is dissolved in the water. The two phases are emulsified using standard pharmaceutical technology and stabilised by the phosphatidyl choline and Pluronic F 68. The amount of morphine sulphate may be altered to provide a range of potencies. A 2ml bolus intravenous injection may be administered every four hours, or the formulation may be incorporated into the reservoir of an analgesic self-administration device.

| | | 1g =~ 1ml |
|---|---|---|
| (b) | MK-329 (a)<br>Fentanyl citrate (b)<br>Soya bean oil (i)<br>Safflower oil (i)<br>Phosphatidyl choline (emulsifier)<br>Glycerine<br>Water (qs 1 ml) (ii)<br>qs 0.1 N NaOH to adjust pH to 7.0 - 7.5 | 0.0015g<br>0.0024g<br>0.0993g<br>0.0993g<br>0.0125g<br>0.0200g<br>0.7665g |

The ingredients were mixed together and emulsified in a similar manner to that described above in Example 1(a).

Example 2 : Intravenous Infusions

| | | |
|---|---|---|
| (a) | MK-329 (a)<br>Morphine sulphate (b)<br>Cottonseed oil (i)<br>Polysorbate (surfactant)<br>Fractionated egg phospholipids (emulsifier)<br>Hydroxypropyl methylcellulose (ii)<br>Water (ii) (adjusted to pH 7 to 7.5 and made isotonic with sorbitol) | 0.015g<br>0.100g<br>200ml<br>1 .6g<br>12.000g<br>5.000g<br>1000ml |

The MK-329 is dispersed in the oil phase and the fentanyl citrate is dispersed in the aqueous phase. The emulsion is formed using standard pharmaceutical techniques. One litre of emulsion may be adminstered intravenously over a 24-hour period. The amount of morphine may be adjusted to allow a range of doses, depending on the response of individual patients.

| | | |
|---|---|---|
| (b) | L-365,260 (a)<br>Morphine sulphate (b)<br>Soya bean oil (i)<br>Egg yolk lecithin (emulsifier)<br>Glycerine (i)<br>Gelatine (ii)<br>Water q.s. to (ii) | 0.03g<br>200mg<br>100g<br>12g<br>25g<br>50 g<br>1 litre |

All the ingredients are emulsified together, except the gelatine, at 80°C. The temperature is reduced to about 40°C and the gelatine added.

Example 3 : S.R. Suppository

| | |
|---|---|
| Butorphenol tartrate (b)<br>MK-329 (a)<br>Hydroxypropyl methylcellulose (ii)<br>Aerosil R972* (thickener)<br>Witepsol H15 or ((i), glycerides)<br>Witepsol W25 to | 30mg<br>12mg<br>300mg<br>100mg<br>2500mg |

(*Hydrophobic silica)

The MK329 and Aerosil are added to molten Witepsol. The butorphenol is blended with hydroxypropyl methylcellulose and then added to the Witepsol mixture. The mixture is poured into 3 ml moulds and shock cooled to room temperature.

Example 4 : Coated S.R. Tablet

| | | |
|---|---|---|
| (a) | Core | |
| | MK-329 (a) | 0.015g |

(continued)

| | | |
|---|---|---|
| (a) | Core | |
| | Suppocire DM (((i), glyceride*)<br>Levorphanol tartrate (b)<br>Crosslinked polyvinyl pyrrolidone (PVP) (ii)<br>Lactose (ii) | 0.100g<br>0.006g<br>0.010g<br>0.175g |
| | Coating | |
| | Cellulose acetate | 0.020g |
| (b) | Core | |
| | Dihydrocodeine tartrate (b)<br>Suppocire DM ((i), glyceride)<br>L-741,528 (a)<br>Polyvinylpyrrolidone (PVP) (ii)<br>Lactose (ii)<br>Magnesium stearate (lubricant) | 180mg<br>100mg<br>4mg<br>40mg<br>123mg<br>1.5mg |

*Suppocire DM is a mixture of hemi-synethetic glycerides of $C_{12-18}$ saturated with fatty acids.

Coating

| | |
|---|---|
| Hydroxypropyl methylcellulose (ii) | 14.45mg |
| Triethyl citrate (plasticiser) 6.9mg | 17.21mg |
| 30% aqueous dispersion ethyl cellulose | |
| Purified water | q.s. |

The dihydrocodeine tartrate is dispersed in molten Suppocire which is cooled with constant stirring to give a granular product. These granules are blended with other materials and tableted.

Example 5 : S.R. Tablet

| | |
|---|---|
| L-740,093 (a) | 0.002g |
| Suppocire DM ((i), glyceride) | 0.100g |
| Dihydrocodeine tartrate (b) | 0.180g |
| Magnesium stearate (lubricant) | 0.003g |
| Hydroxypropyl methyl cellulose (ii) | 0.250g |
| | 0.548g |

The L-740,093 is dissolved in molten Witepsol W25 at 50-60°C. The resulting liquid is atomised into a chamber containing chilled nitrogen (gas) at about 10°C. Spherical particles produced thereby are in the range 80-120 µm. These are then blended with the dihydrocodeine tartrate and remaining excipients to produce a powder which is tabletted by standard techniques.

Example 6 : S.R. Capsules

| | |
|---|---|
| L-369,466 (a) | 0.0004g |
| Morphine sulphate (b) | 0.100g |
| Suppocire DM ((ii), glyceride) | 0.200g |
| Sodium alginate (i) | 0.200g |

The L-369,466 and morphine are incorporated into molten Witepsol. Granules or spheroids are produced by standard pharmaceutical techniques, then blended with the sodium alginate before filling into capsule shells. The amount of morphine in each capsule, for once daily administration, can be changed within the range 30mg to 150mg.

## Claims

**1.** A pharmaceutical formulation comprising

(a) an opioid-potentiating amount of a CCK antagonist;
(b) an analgesic amount of an opioid; and
(c) a pharmaceutically acceptable biphasic carrier comprising

(i) an organic phase comprising a glyceride derivative; and
(ii) a hydrophilic phase.

**2.** A pharmaceutical formulation according to claim 1, wherein the organic phase (i) has a solubilising capacity for the CCK antagonist in excess of 5mg per gram of organic phase.

**3.** A pharmaceutical formulation according to claim 1 or 2, wherein the organic phase comprises an oil selected from soya bean, safflower, sesame, rapeseed, peanut, olive, cotton seed and fish oils, alone or in combination with glycerine and/or a wax selected from full and/or partial triglycerides of fatty acids.

**4.** A pharmaceutical formulation according to any one of claims 1 to 3, intended for intravenous use, wherein the hydrophilic phase is aqueous and has a viscosity of from 2500-7500cp at 20°C.

**5.** A pharmaceutical formulation according to any one of claims 1 to 3, intended for use as a solid formulation, wherein the hydrophilic phase is gel forming, incorporates the opioid in the gel and forms a matrix incorporating the CCK antagonist and the glyceride derivative.

**6.** A pharmaceutical formulation according to any one of the preceding claims wherein the hydrophilic phase comprises a pharmacologically and pharmaceutically acceptable polymer or salt thereof selected from proteins such as gelatine, hyaluronic acid, alginic acids or salts thereof such as sodium alginate, carboxymethylcellulose (optionally cross-linked), methyl cellulose, other cellulose derivatives which are water-swellable such as hydroxypropylmethylcellulose and hydroxyethyl-cellulose or other water-swellable polymers such as polyvinyl pyrrolidone (PVP) or water-soluble polymers such as lactose.

**7.** A pharmaceutical formulation according to any one of the preceding claims, wherein the carrier is in the form of an oil-in-water emulsion.

**8.** A pharmaceutical formulation according to claim 7, wherein the oil-in-water emulsion comprises

(i) an oil phase comprising a glyceride derivative; and
(ii) an aqueous phase optionally comprising a buffer whereby the emulsion has a pH of from 6.5 to 7.5 and optionally comprises an isotonicity regulator whereby the aqueous phase is made isotonic to blood plasma.

**9.** A pharmaceutical formulation according to claim 7 or 8 wherein the average particle size of the emulsion is from 0.2 to 3.0μm.

**10.** A pharmaceutical formulation according to any one of claims 7 to 9 further comprising en emulsifying agent, a surfactant and/or a pH adjuster.

**11.** A pharmaceutical formulation according to any one of the preceding claims, wherein the CCK antagonist (a) has been incorporated into the organic phase (i) and the opioid analgesic (b) has been incorporated into the hydrophilic phase (ii).

**12.** A pharmaceutical formulation according to any one of the preceding claims, wherein the ratio of component (i) to component (ii) is within the range of 10:1 to 1:5 by weight.

**13.** A pharmaceutical formulation according to any one of the preceding claims, wherein the ratio of component (a) to component (b) is within the range of 1:2 to 1:40 by weight.

**14.** A pharmaceutical formulation according to any one of the preceding claims, wherein the CCK antagonist (a) is selected from:

3S-(-)-(1,3-dihydro-3-(2-indolecarbonylamino)-1-methyl-5-phenyl-2H-1,4-benzodiazepin-2-one;
3R-3-(N'-(3-methylphenyl)ureido)-1,3-dihydro-1-methyl-5-phenyl-2H-1,4-benzodiazepin-2-one;
N-[1,3-dihydro-1-methyl-2-oxo-5-phenyl-1H-1,4-benzodiazepin-3-yl]-N'-[3-(1,2,4-oxodiazol-5-one)phenyl] urea;
(-)-N-[2,3-dihydro-5-(4,4-dimethylpiperidin-1-yl)-1-methyl-2-oxo-1H-1,4-benzodiazepin-3-yl]-N'-[indan-5-yl] urea; and
[N-[(3R)-5-[3-azabi-cyclo[3.2.2]nonan-3-yl)-2,3-dihydro-1-methyl-2-oxo-1H-1,4-benzodiazepin-3-yl] -N'-(3-methylphenyl)urea].

**15.** A pharmaceutical formulation according to any one of the preceding claims, wherein the opioid (b) is selected from morphine, codeine, or a salt thereof, and 14-hydroxymorphinan opioid analgesics and salts thereof.

**16.** A pharmaceutical formulation according to any one of the preceding claims as a solid formulation, an injectable emulsion, suppositories or tablets.

**17.** A pharmaceutical formulation according to any one of the preceding claims in unit dosage form suitable for the delivery of 0.5 to 300mg per day of CCK antagonists to a patient in need thereof.

**18.** A pharmaceutical formulation according to claim 17 in unit dosage form suitable for oral use or use as a suppository for the delivery of 1 to 100mg per day of CCK antagonist to a patient in need thereof.

**19.** A pharmaceutical formulation according to claim 17 in unit dosage form suitable for intravenous use for the delivery of 1 to 300 mg per day of CCK antagonist to a patient in need thereof.

**Patentansprüche**

**1.** Pharmazeutische Formulierung, enthaltend

(a) eine opioidpotenzierende Menge eines CCK-Antagonisten;
(b) eine analgetische Menge eines Opioids; und
(c) einen pharmazeutisch verträglichen, biphasischen Träger, der

(i) eine organische Phase, die ein Glyceridderivat enthält; und
(ii) eine hydrophile Phase enthält.

**2.** Pharmazeutische Formulierung nach Anspruch 1, bei der die organische Phase (i) eine Solubilisierungskapazität für den CCK-Antagonisten von mehr als 5 mg pro Gramm der organischen Phase hat.

**3.** Pharmazeutische Formulierung nach Anspruch 1 oder 2, bei der die organische Phase ein Öl, ausgewählt aus Soja-, Distel-, Sesam-, Raps-, Erdnuss-, Oliven-, Baumwollsamen- und Fischölen, allein oder in Kombination mit Glycerin und/oder Wachs, ausgewählt aus vollständigen oder partiellen Triglyceriden von Fettsäuren, enthält.

4. Pharmazeutische Formulierung nach einem der Ansprüche 1 bis 3, die zur intravenösen Verwendung vorgesehen ist, wobei die hydrophile Phase wässrig ist und eine Viskosität von 2500 bis 7500 cp bei 20°C aufweist.

5. Pharmazeutische Formulierung nach einem der Ansprüche 1 bis 3, die zur Verwendung als feste Formulierung vorgesehen ist, wobei die hydrophile Phase gelbildend ist, das Opioid in das Gel einschließt und eine Matrix bildet, die den CCK-Antagonisten und das Glyceridderivat einschließt.

6. Pharmazeutische Formulierung nach einem der vorhergehenden Ansprüche, bei der die hydrophile Phase ein pharmakologisch und pharmazeutisch verträgliches Polymer oder Salz davon, ausgewählt aus Proteinen, wie Gelatine, Hyaluronsäure, Alginsäuren oder Salzen davon, wie Natriumalginat, Carboxymethylcelluluse (gegebenenfalls vernetzt), Methylcellulose, anderen Cellulosederivaten, die wasserquellbar sind, wie Hydroxypropylmethylcellulose und Hydroxyethylcellulose, oder anderen wasserquellbaren Polymeren, wie Polyvinylpyrrolidon (PVP), oder wasserlöslichen Polymeren, wie Lactose, enthält.

7. Pharmazeutische Formulierung nach einem der vorhergehenden Ansprüche, bei der der Träger in Form einer Öl-in-Wasser-Emulsion vorliegt.

8. Pharmazeutische Formulierung nach Anspruch 7, bei der die Öl-in-Wasser-Emulsion

(i) eine Ölphase, die ein Glyceridderivat enthält; und
(ii) eine wässrige Phase enthält, die gegebenenfalls einen Puffer enthält, wodurch die Emulsion einen pH-Wert von 6,5 bis 7,5 aufweist, und gegebenenfalls ein Isotonizitätsregulierungsmittel enthält, wodurch die wässrige Phase isoton gegenüber Blutplasma gemacht wird.

9. Pharmazeutische Zusammensetzung nach Anspruch 7 oder 8, bei der die durchschnittliche Teilchengröße der Emulsion 0,2 bis 3,0 μm beträgt.

10. Pharmazeutische Formulierung nach einem der Ansprüche 7 bis 9, die ferner ein Emulgierungsmittel, ein Tensid und/oder ein pH-Einstellungsmittel enthält.

11. Pharmazeutische Formulierung nach einem der vorhergehenden Ansprüche, bei der der CCK-Antagonist (a) der organischen Phase (i) einverleibt worden ist, und das Opioidanalgetikum (b) der hydrophilen Phase (ii) einverleibt worden ist.

12. Pharmazeutische Formulierung nach einem der vorhergehenden Ansprüche, bei der das Verhältnis von Komponente (i) zu Komponente (ii) im Bereich von 10:1 bis 1:5 liegt, bezogen auf das Gewicht.

13. Pharmazeutische Formulierung nach einem der vorhergehenden Ansprüche, bei der das Verhältnis von Komponente (a) zu Komponente (b) im Bereich von 1:2 bis 1:40 liegt, bezogen auf das Gewicht.

14. Pharmazeutische Formulierung nach einem der vorhergehenden Ansprüche, bei der der CCK-Antagonist (a) ausgewählt ist aus:

3S-(-)-(1,3-Dihydro-3-(2-indolcarbonylamino)-1-methyl-5-phenyl-2H-1,4-benzodiazepin-2-on;
3R-3-(N'-3-Methylphenyl)ureido)-1,3-dihydro-1-methyl-5-phenyl-2H-1,4-benzodiazepin-2-on;
N-[1,3-Dihydro-1-methyl-2-oxo-5-phenyl-1H-1,4-benzodiazepin-3-yl]-N'-[3-(1,2,4-oxodiazol-5-on)phenyl] harnstoff;
(-)-N-[2,3-Dihydro-5-(4,4-dimethylpiperidin-1-yl)-1-methyl-2-oxo-1H-1,4-benzodiazepin-3-yl)-N'-(indan-5-yl] harnstoff und
[N-[(3R)-5-(3-Azabicyclo[3.2.2]nonan-3-yl)-2,3-dihydro-1-methyl-2-oxo-1H-1,4-benzodiazepin-3-yl]-N-(3-methylphenyl)harnstoff].

15. Pharmazeutische Formulierung nach einem der vorhergehenden Ansprüche, bei der das Opioid (b) ausgewählt ist aus Morphin, Codein oder einem Salz davon sowie 14-Hydroxymorphinan-Opioidanalgetika und Salzen davon.

16. Pharmazeutische Formulierung nach einem der vorhergehenden Ansprüche als feste Formulierung, injizierbare Emulsion, Zäpfchen oder Tabletten.

17. Pharmazeutische Formulierung nach einem der vorhergehenden Ansprüche in Einzeldosisform, die zur Abgabe von 0,5 bis 300 mg CCK-Antagonist pro Tag an einen Patienten geeignet ist, der dessen bedarf.

18. Pharmazeutische Formulierung nach Anspruch 17 in Einzeldosisform, die zur oralen Verabreichung oder zur Verwendung als Zäpfchen zur Abgabe von 1 bis 100 mg CCK-Antagonist pro Tag an einen Patienten geeignet ist, der dessen bedarf.

19. Pharmazeutische Formulierung nach Anspruch 17 in Einzeldosisform, die zur intravenösen Verabreichung zur Abgabe von 1 bis 300 mg CCK-Antagonist pro Tag an einen Patienten geeignet ist, der dessen bedarf.

**Revendications**

1. Formulation pharmaceutique comprenant :

   (a) une quantité d'un antagoniste de la CCK potentialisant un opioïde ;
   (b) une quantité analgésique d'un opioïde ; et
   (c) un véhicule biphasique pharmaceutiquement acceptable comprenant :

      (i) une phase organique comprenant un dérivé de glycéride ; et
      (ii) une phase hydrophile.

2. Formulation pharmaceutique selon la revendication 1, dans laquelle la phase organique (i) présente une capacité de solubilisation pour l'antagoniste de la CKK en excès de 5 mg par gramme de phase organique.

3. Formulation pharmaceutique selon la revendication 1 ou 2, dans laquelle la phase organique comprend une huile choisie parmi les huiles de soja, de carthame, de sésame, de colza, d'arachide, d'olive, de coton et de poisson, seule ou en association avec de la glycérine et/ou une cire choisie parmi les triglycérides entiers et/ou partiels d'acides gras.

4. Formulation pharmaceutique selon l'une quelconque des revendications 1 à 3, destinée à une utilisation intraveineuse, dans laquelle la phase hydrophile est aqueuse et présente une viscosité d'environ 2500 à 7500 cp à 20°C.

5. Formulation pharmaceutique selon l'une quelconque des revendications 1 à 3, destinée à une utilisation en tant que formulation solide, dans laquelle la phase hydrophile forme un gel, incorpore l'opioïde dans le gel et forme une matrice incorporant l'antagoniste de la CCK et le dérivé de glycéride.

6. Formulation pharmaceutique selon l'une quelconque des revendications précédentes, dans laquelle la phase hydrophile comprend un polymère pharmacologiquement et pharmaceutiquement acceptable, ou un sel de celui-ci, choisi parmi les protéines telles que la gélatine, l'acide hyaluronique, les acides alginiques, ou des sels de ceux-ci, tels que l'alginate de sodium, la carboxyméthylcellulose (éventuellement réticulée), la méthylcellulose, d'autres dérivés de la cellulose qui sont capables de gonfler dans l'eau tels que l'hydroxypropylméthylcellulose et l'hydroxyéthylcellulose ou d'autres polymères capables de gonfler dans l'eau tels que la polyvinylpyrrolidone (PVP) ou des polymères solubles dans l'eau tels que le lactose.

7. Formulation pharmaceutique selon l'une quelconque des revendications précédentes, dans laquelle le véhicule est sous la forme d'une émulsion huile dans eau.

8. Formulation pharmaceutique selon la revendication 7, dans laquelle l'émulsion huile dans eau comprend :

   (i) une phase huileuse comprenant un dérivé de glycéride ; et
   (ii) une phase aqueuse comprenant éventuellement un tampon au moyen duquel l'émulsion a un pH de 6,5 à 7,5, et comprend éventuellement un régulateur d'isotonicité au moyen duquel la phase aqueuse est rendue isotonique par rapport au plasma sanguin.

9. Formulation pharmaceutique selon la revendication 7 ou 8, dans laquelle la taille moyenne des particules de l'émulsion est de 0,2 à 3,0 µm.

**10.** Formulation pharmaceutique selon l'une quelconque des revendications 7 à 9, comprenant en outre un agent émulsifiant, un tensio-actif et/ou un régulateur de pH.

**11.** Formulation pharmaceutique selon l'une quelconque des revendications précédentes, dans laquelle l'antagoniste de la CCK (a) a été incorporé dans la phase organique (i) et l'opioïde analgésique (b) a été incorporé dans la phase hydrophile (ii).

**12.** Formulation pharmaceutique selon l'une quelconque des revendications précédentes, dans laquelle le rapport entre le composant (i) et le composant (ii) est compris dans la gamme de 10:1 à 1:5 en masse.

**13.** Formulation pharmaceutique selon l'une quelconque des revendications précédentes, dans laquelle le rapport entre le composant (a) et le composant (b) est compris dans la gamme de 1:2 à 1:40 en masse.

**14.** Formulation pharmaceutique selon l'une quelconque des revendications précédentes, dans laquelle l'antagoniste de CCK (a) est choisi parmi :

la 3S-(-)-(1,3-dihydro-3-(2-indolecarbonylamino)-1-méthyl-5-phényl-2H-1,4-benzodiazépin-2-one ; la 3R-3-(N'-(3-méthylphényl)uréido)-1,3-dihydro-1-méthyl-5-phényl-2H-1,4-benzodiazépin-2-one ; la N-[1,3-dihydro-1-méthyl-2-oxo-5-phényl-1H-1,4-benzodiazépin-3-yl]-N'-[3-(1,2,4-oxodiazol-5-one)phényl]urée ; la (-) -N-[2,3-dihydro-5-(4,4-diméthylpipéridin-1-yl)-1-méthyl-2-oxo-1H-1,4-benzodiazépin-3-yl]-N'-[indan-5-yl] urée; et la [N-[(3R)-5-(3-azabi-cyclo[3.2.2]nonan-3-yl)-2,3-dihydro-1-méthyl-2-oxo-1H-1,4-benzodiazépin-3-yl]-N'-(3-méthylphényl )urée].

**15.** Formulation pharmaceutique selon l'une quelconque des revendications précédentes, dans laquelle l'opioïde (b) est choisi parmi la morphine, la codéine ou des sels de celles-ci, et des analgésiques opioïdes 14-hydroxymorphinan et des sels de ceux-ci.

**16.** Formulation pharmaceutique selon l'une quelconque des revendications précédentes, sous forme d'une formulation solide, d'une émulsion injectable, de suppositoires ou de comprimés.

**17.** Formulation pharmaceutique selon l'une quelconque des revendications précédentes, sous forme de dose unitaire, adaptée pour la délivrance de 0,5 à 300 mg par jour d'antagonistes de la CCK à un patient le nécessitant.

**18.** Formulation pharmaceutique selon la revendication 17, sous forme de dose unitaire, adaptée pour l'utilisation orale ou l'utilisation comme suppositoire pour la délivrance de 1 à 100 mg par jour d'antagoniste de la CCK à un patient le nécessitant.

**19.** Formulation pharmaceutique selon la revendication 17, sous forme de dose unitaire, adaptée pour l'utilisation intraveineuse pour la délivrance de 1 à 300 mg par jour d'antagoniste de CCK à un patient le nécessitant.